# EUROPEAN PATENT APPLICATION

(11) **EP 3 716 283 A1**
(43) Date of publication of application: **30.09.2020**
(21) Application number: 19166250.1
(22) Date of filing: 29.03.2019
(51) Int. Cl.: G16H 40/63

(54) **CONTROL OF DIALOG WINDOWS IN A MULTI-APPLICATIONAL MEDICAL SETTING**

(71) Applicant: Siemens Healthcare GmbH, 91052 Erlangen (DE)
(72) Inventor: Charrad, Chiheb, 91077 Neunkirchen A. Brand (DE); Schneider, Roland, 91056 Erlangen (DE)

(57) **Abstract**

The invention relates to a method and related devices for controlling a positioning of windows (DW, PMW, SMW, OW) with respect to a z-order for at least one application (A1, A2), running on a computer with a monitor system (M1, M2, M3), wherein the at least one application (A1) has a primary main window (PMW) and a secondary main window (SMW), wherein the primary main window (PMW) may own a set of dialog windows (DW). After an activation signal is received on a window, messages directed to the main windows (PMW; SMW) are detected and amended by creating different control signals for instructing the positioning of the windows in each case.

## Description

The invention generally relates to the field of graphical user interfaces and in particular to positioning or arranging windows on a monitor system.

Most operating systems in use today, e.g. Windows, Linux etc., operate using the window technique (windowing), which allows a user to work on different applications at the same time and to control them using the user interface.

Further, special fields of engineering, such as medical engineering, require that a relatively large group of people be able to work with an application. These include, by way of example, scenarios for obtaining results for a patient using one or more imaging applications. In this case it is preferred to work with a set of different applications in parallel. In this context it makes sense for particular windows of the respective applications to be distributed, positioned and displayed on a plurality of monitors.

Since a plurality of applications are usually executed in parallel, that is to say at the same time, a plurality of windows normally need to be displayed on a monitor system. An application may have more than one main application window. The application may produce at set of dialog windows which are related to a main window by an owner relation or parent relation. The relation may be either directly or indirectly through a cascading chain of the owner (or parent) relation. The dialog window is a property or message window of any type in a respective application. These include for example popups with an input request or popups with important warnings, which need instant attention.

To ensure a smooth operating sequence which is as optimum as possible for a plurality of applications and hence for a plurality of main windows on a monitor, a particular method for positioning the various windows on a monitor is known for systems from the prior art. This positioning mechanism is intended, in particular, to ensure that a dialog window in an application is always visible to the user and hence is opened over the main window of the respective application. This makes it possible to ensure that the user actually sees the data displayed in the dialog window (e.g. the warnings) immediately. In addition, this makes it possible to ensure that all dialog windows are positioned visibly on the monitor without any further activity by the user.

In the Microsoft WINDOWS operating systems and derivatives thereof, this positioning mechanism is based on a "Z-order". In similar fashion to the mathematical Z axis in a coordinate system, the respective windows in the applications are arranged on an imaginary line which runs at right angles to the monitor. The windows are arranged on a stack, so to speak, with the first element of the stack also appearing at the topmost position on the monitor and hence always being visible, while the last element of the stack is arranged right at the back in the Z-order and it could be hidden by the windows positioned in front of it. Each window which is to be displayed on the monitor has a unique position within this Z-order. Depending on the arrangement in this Z-order, a window may be fully visible or fully or partly hidden by others. Generally, a window may refer to a user interface to be included, e.g. control elements such as buttons, scrollbars etc. In Windows-based systems, a "Window Manager" controls the arrangement of the windows. Two separate sections are defined in the Z-order: a topmost area and a normal area. In the topmost area, all windows, situated in this area are guaranteed to be displayed in front of all other windows and are thus always visible. By contrast, the windows in the normal area may be hidden by other windows. The concept of the Z-order is derived from a predecessor/successor hierarchy and defines a "before/after" or an "above/below" arrangement. All the windows on a monitor are therefore depicted within this Z-order hierarchy. This hierarchy starts at the desktop window as root window. As the direct successors or children of the desktop window, a plurality of main windows may be arranged on a "top-level window level". A respective one of these main windows can then again be split into one or more child windows, in a manner of a tree. These child windows may be popup windows or other child windows. A child window can have only one main window as predecessor.

A further complexity arises for multi-monitor-systems, in which the applications with its generated windows may be distributed over a plurality of monitors. Also, in multi-monitor-settings a correct positioning of windows has to be assured.

In this respect it is referred to DE 10 2005 011 670 B4 in which a further positioning mechanism is suggested in order to arrange the windows on an additional monitor in a multi-monitor-setting. This document suggests moving the main window of the amended application always at the bottommost z-order position of all applications running on the system which ensures that the dialogs of the amended application appear in front of the main windows of this application, however in the case other applications are present and overlap the amended application, these will cover the amended applications main windows. As a cure for these problems this document suggests manipulating the Z-order of the operating system. This, however, is an essential risk, especially for medical applications, because by changing the operating system's behavior, failures and unintended side effects may occur.

Further, this document does not or not satisfactorily address potential overlapping and hiding problems of dialog windows which originate from an application which has more than one main window, which is a typical setting nowadays. If for example a dialog window of a primary main window is moved by user interaction in front of another secondary main window of the same application, hiding problems may arise, so that e.g. a dialog window may be at least partially hidden by the secondary main window or by other windows of third-party applications. Further, the mechanism of the state of the art systems do not address the problem of correct window positioning (without hiding important information in other colliding windows) if the application has more than one main window or if the main window does not have an owner relation and should be moved in the z-order or its dialog windows should be moved.

As medical technology is evolving fast, the typical situation nowadays is, that on the one hand more applications, which generate more than one main window need to be operated in parallel and on the other hand that large scale monitors may be used in order to display windows of a set of applications on one common monitor.

In previously known systems, one problem is that an interference with the well established operating systems' behavior is necessary, in particular a manipulation of the Z-order is executed. This means a high risk, that proper display of dialog windows may not be assured also in unusual complex settings. Further, in previously known systems, the overlapping problem has not been solved to date for applications which generate more than one main window with corresponding dialog windows.

It is therefore an object of the present invention to provide a solution for the above mentioned problems. Further, it is an object to improve the control of displaying windows without impeding security. Finally, the operating system and its window arrangement functionality (e.g. Z-order of other applications) should not be changed.

This object is solved by a method for controlling a positioning of a set of windows, by a z-order adaption module, by a computer program according to the appended independent claims. Advantageous aspects, features and embodiments are described in the dependent claims and in the following description together with advantages.

In the following the solution according to the invention is described with respect to the claimed method. Features, advantages or alternative embodiments mentioned with respect to the method may also be applied to the device-type claims (e.g. the z-order adaption module) and vice versa, respectively.

According to a first aspect the invention relates to a computer implemented method for controlling a positioning of more than one window with respect to a z-order for at least one application, running on a computer with a monitor system, wherein the at least one application has a primary main window and at least one secondary main window, wherein the primary main window may own a set of dialog windows, wherein the method reacts on the request to bring any of the multitude of windows to the foreground of the monitor system with a request to bring the dialog windows and all main windows to the foreground, thereby ensuring that the dialog windows are always in front of any of the primary and secondary main windows, and wherein the z-order of the primary and secondary main windows can change within the application, which ensures that the z-order of overlapping primary and secondary main windows can be altered in a way that its content can be made visible and wherein primary and secondary main windows of the application are contiguous within a desktop window list which ensures that no other window of any other application appears mixed with the application. Generally, a window list (desktop windows list or top-level window list) is maintained and published by a window positioning mechanism of the operating system, e.g. WINDOWS.

In a preferred embodiment the method comprises:
- Receiving an activation signal (which may relate to a request to evaluate whether the z-order has to be changed) on a window (or on an element or portion thereof) - so that the very window becomes an activated window. The activation signal may be received via e.g. user interaction (click, key stroke);
- Detecting whether the activation signal was received on a dialog window owned by the primary main window, the primary main window itself or on a (or on one of the) secondary main window(s);
- If the activation signal was received on a dialog window: Issue a first control signal to let the operating system's positioning mechanism execute and detect a change request for the primary main window and within the primary main window change request: insert all secondary main windows before or after the primary main window so that the order of main windows is retained.
- If the activation signal was received on the primary main window:
   Within the primary main window change request: insert all secondary main windows after the primary main window so that the order of all secondary main windows is retained and the primary main window is on top of the secondary main windows. (please note: No second control signal is necessary because for the primary main window the dialogs are properly positioned by means of the owner relation to the primary main window which is the standard behavior of the operating system);
- If the activation signal was received on one of the at least one secondary main windows:
   Issue a third control signal for:
   - Detecting messages or instruction signals from the operating system's positioning mechanism to the secondary main window,
   - Triggering the primary main window to be moved where the secondary main window is requested (i.e. to the front) which moves all dialog windows to the front according to the operating system's positioning mechanism and for
   - Amending the original secondary main window instruction signal so that the secondary main window is moved in front of the primary main window and after the dialog windows and insert all remaining secondary main windows such that the overall z-order of all main windows except the notified secondary main window is retained.

The method takes advantage of the standard operating system's behavior that windows of third-party applications automatically 'fall' behind the windows of the amended application, without actively amending the code of the third-party application and without amending the operating system.

The method comprises that the messages exchanged between the z-order adaption module for executing the method described above and the operating system or its positioning mechanism are analyzed and intercepted.

The method, mentioned above, may be provided as a computer-implemented algorithm. According to another aspect, the present invention relates to applying the algorithm in a system with a set of (i.e. medical) applications.

The core idea of the present invention is to provide a window positioning mechanism which works on an applicational level and not on a superordinate or global level, e.g. not on the level of the operating system and without amending the operating's positioning process (z-order mechanism). This has the technical effect that the application may be deployed and operated with default operating system privileges and does not require administrative privileges which are required for changing operating system functionality. The proposed solution is simple, functional and may even be applied in complicated scenarios with applications with more than one secondary main window.

The core idea is that a "natural" behavior of window positioning which is known for an application with one single main window is to be transferred to an application with more than one main window. In the "one main window setting" the following appearance features or characteristics do apply:
1. All dialog windows of the application are to be placed in front of the main window.
2. If a user interaction is received on one of the windows of the application (being the main window or one of the dialog windows), then all the windows of the application will be displayed in common and be brought simultaneously in the foreground and in front of windows of other applications.
3. There is no window of another application which is positioned in between the cascade of windows of the application.
4. The cascade of windows of an application (so called nested dialogs) is reflected by an owner relation (WINDOWS based systems) or parent relation (for UNIX based systems). The main window owns dialog windows, which in turn may own further dialog windows and so on and so forth.

According to the invention, the above mentioned features do also apply to an application with several main windows. The cascade of windows which in complex applications may have a tree structure is reflected in the "owned by" relation - a dialog window is owned by its owner (or parent) window, which may be a dialog window as well or may be a primary main window. The control of the windows for the application is achieved by providing and using a list with all main windows of the application. This list may be stored and administered by a z-order adaption module or an associated storage thereof. The list of main windows is application local. The list is preferably sorted by the actual z-order of the main windows in order to be able to retain the order of MWs during operation of the method and to restore the z-order after e.g. a preceding minimizing request.

Thus, in a preferred embodiment, individual lists with all main windows for each application are generated and processed.

The situation for positioning windows may become challenging if more applications are executed in parallel and if all or some of these applications do have more than one main window. By means of the solution suggested herein, conflicting scenarios for window positioning in these situations are dissolved in a secure manner based on the amended application not affecting other applications, so that medical standard requirements may be met.

In a preferred embodiment, the method may be executed as an instance which is integrated in the application or used by it. Execution of the method causes to intercept the messages which are sent from the application (each of them) to the positioning mechanism of the operating system in order to generate an adapted entry in a z-order list of the operating system without changing the operating system as such.

According to another preferred embodiment, generating the second control signal causes dialog windows to be moved to the front in the z-order although they are not related by the owner relation with the corresponding main window (being the secondary main window) which has been activated. This has the advantage that the natural appearance of the windows is not impaired and that the application may be operated as efficient as possible (without waiting times for manual changing the ordering of the windows due to overlapping).

According to another preferred embodiment, if the monitor system is a single monitor system, e.g. a large scale monitor with high resolution, all windows of different applications are to be displayed simultaneously. Alternatively, if the monitor system is a multi-monitor system, the windows may be distributed over several monitors. Especially in this scenario, it has to be assured and it is assured that all dialog windows of the active application are not hidden by any other windows. The technical parameters of the monitors of the multi-monitor-setting may even differ without affecting the effectiveness of the positioning mechanism. The feature, that the method and module according to this invention may be used on both of the settings has the advantage that it may be applied more extensively.

According to another preferred embodiment, the activation signal is effected by a user interaction on a window (or another type of graphical user interface element, like a bar or a button) and/or by an activation of at least one key and/or by a functional request of an application transmitted via an application programming interface/API. This improves flexibility of the method.

According to another preferred embodiment, at least one third-party application (also called 'other application') is hosted within the application, and wherein the third-party application has at least one main window (also called 'other window'). The main (other) window of the third-party application is hosted by adding them as child windows to main windows of the hosting application. Dialog windows (other windows) of the third-party application are detected and added as dialog windows to the hosting application's primary main window. This is an important technical feature because it is possible to apply the positioning mechanism also to such hosted third-party applications. This setting is more challenging, because the code of the third-party application cannot be adapted. However, nevertheless the object of a correct positioning may be achieved for all windows of the set of applications, including third-party applications, which is a major advantage since such hosted applications from different manufacturers can be integrated seamless without access to the source code of the third-party application for amending the same. The integration of such a third-party application (with its other window(s)) is executed by detecting its main window after having started the third-party application and embedding the detected main window in the hosting application at an appropriate position in the layout. For integrating dialog windows of the third-party application, a window creation detection system can be implemented based on the operating system's application programming interfaces (APIs), which is adapted to detect all dialog windows of the very third-party application and amend the original owner of the detected dialogs by changing it to the primary main window of the hosting application. This is achieved because the hosting application starts the hosted application so that the application process is known. This in turn allows that the detection of the windows may be implemented with standard operating system's interfaces (APIs).

According to another preferred embodiment, the positioning even works if at least one dialog window is moved by user interaction out of its original lateral or x-/y-position and at least in part out of a region of its primary main window. For example, it may be moved in front of the secondary main window.

According to another preferred embodiment, although the primary main window and the secondary main window and optionally other windows may at least partially overlap or may be displayed on separate monitor devices of the monitor system (which may e.g. be a multi-monitor system), it is assured that the dialog windows are displayed at a z-order (position) so that they are always fully visible whenever any of the windows of the amended application is activated. The mechanism ensures that all main and dialog windows are ordered according to a contiguous z-order range, so that all application windows are displayed in a compact, coherent and correct manner. Doing so, the mechanism makes it impossible for windows of other applications to disrupt the natural window order of the amended application. Since the mechanism only affects the ordering of windows owned by the amended application, it does not influence the behavior of other applications, so that there is no need to change the operating system's behavior.

In a preferred embodiment, the z-order of the main windows of the application is changed in reply to where the activation signal was detected, in particular on which window.

Up to now, the invention has been described with respect to the claimed method. Features, advantages or alternative embodiments herein can be assigned to the other claimed objects (e.g. the computer program or a z-order adaption module) and vice versa. In other words, the subject matter which is claimed or described with respect to the device can be improved with features described or claimed in the context of the method and vice versa. In this case, the functional features of the method are embodied by structural units of the system and vice versa, respectively. Generally, in computer science a software implementation and a corresponding hardware implementation are equivalent. Thus, for example, a method step for "storing" data may be performed with a storage unit and respective instructions to write data into the storage. For the sake of avoiding redundancy, although the device (module) may also be used in the alternative embodiments described with reference to the method, these embodiments are not explicitly described again for the device.

In another aspect, the present invention relates to a z-order adaption module, wherein an instance of the z-order adaption module may be deployed in an application, which generates content, in particular medical content, to be displayed in windows of a monitor system. The z-order adaption module is configured to execute the method for controlling the positioning of windows, as mentioned above. With other words, the instance of the z-order adaption module is adapted to execute the method as described above which in turn effects the application to apply the method and to show the functionality for secure visibility of dialog windows. The z-order adaption module may be integrated in a computer system, which is configured to run the applications. The computer system may be a medical system and may particularly be part of a medical imaging system.

Another aspect therefore relates to a medical computer-based system with such a z-order adaption module.

In another aspect the invention relates to a computer program comprising program elements which induce a computer to carry out the steps of the method for controlling a window positioning according to the method above, when the program elements are loaded into a memory of the computer.

In the following a short definition of terms used within this application is given.

The application is a computer program to be executed on a computing device. The application may be of any functionality but has a graphical user interface to a monitor system for displaying graphical user interface elements like windows, tool bars or buttons or other elements. In particular, the application may be a diagnosis and/or imaging application in the medical domain. The application may be used in a multi-monitor setting.

In particular, two categories of applications are differentiated: A first type of application - mentioned simply as 'application' -, whose the source code is available and a second kind of application - mentioned as third-party application or other application -, where the source code is not available so that its behavior may not be changed by re-coding.

The application, when being executed on the computer, creates different types of windows. Main windows are the windows which are generated as the main user interface and represent a root in a tree structure. A major extension of the present application relates to the fact that an application may have more than one main window - whereas to the contrary in state of the art, an application may create multiple windows but the operating system for the windows positioning processes these applications as having exactly one main window and not more than one. According to this invention, it is therefore differentiated between two different types of main windows: primary main windows and secondary main windows. Primary main windows act as the owner for dialog windows or a cascade of dialog windows; whereas secondary main windows may not own dialog windows. So, a primary main window may own a first dialog window, which in turn may own a second dialog window and so on and so forth. These first, second and further dialog windows are functionally dependent, form a cascade and relate to one common origin (in the tree structure: one branch). That is to say that it is assured that the interrelated dialog windows are always positioned in front of their primary main window and that no (so called 'other') window of other applications may be positioned in between one of these windows in the z-order arrangement according to the state of the art functionality of the positioning mechanism of the operating system. The final result with the z-order positioning of the windows is independent of the x/y position of the primary and secondary main windows on the screen and is further independent of the relative position of the primary and secondary main windows with respect to each other. Further, the positioning of the main windows is independent of the x/y-position of the dialog windows. Moreover, the positioning result is independent of the number of monitors and the placement or distribution of the windows on these monitors.

The positioning method according to the invention has the technical advantage that it works for any number of main windows and for any number of dialog windows. Further, it is not necessary to engage with and adapt the operating systems z-order administration.

A window may be any kind of graphical user interface element, like a screen portion, a tool bar, any type of widget, which represents at least a part of the human machine interface of the application and is controlled by a windowing system, typically comprising a window manager, a display server and the monitor hardware.

According to the invention two different classes or categories of main windows are provided and processed: "primary main windows" and "secondary main windows". By definition, the secondary main window category (in the following abbreviated as secondary main window) does not own one or more dialog windows, and the secondary main window belongs to (or is generated by) the same application as the primary main window. The primary main window category (in the following abbreviated as primary main window) does own one or more dialog windows.

"Positioning" refers to placing windows on a screen or a monitor with respect to their z-order (dimension: in front of or behind of). In case a lateral positioning should be addressed, it is explicitly referred to as x-/y-positioning or lateral positioning. Without such indication, a z-order positioning is meant.

A global window creation detection system may be used, like e.g. the method SetWinEventHook in the WINDOWS operating system.

The z-order adaption module is an instance of a computer program for execution of the above mentioned method. The z-order adaption module is an electronic instance or a digital object and serves as an additional intermediary node between the respective application and the operating system. In particular, it serves to intercept the messages transferred from the operating system, more specifically from the positioning mechanism of the operating system. The z-order adaption module amends the messages in that it changes the instructions for entry in the z-order list.

The method is computer-implemented and processes several input data. The input data comprise:
- the target of the activation signal, i.e. the window, on which the activation signal was received or to which it was addressed (if via API instruction);
- the instructions sent from the operating system to the application;
- The current z-ordered list of all top level windows of all applications as published by the positioning mechanism of the operating system.

The output is a first or second or third control signal for instructing the operating system's window positioning mechanism to position each window of the set of windows of the application. Of course, the method, suggested in herein may be applied to a set of applications which are executed by the operating system in parallel. The first, second and third control signal is in reply to a result of a computer processing (executed by the z-order adaption module), in particular of a distinction of three cases. The first case relates to a reception of the activation signal on a dialog window and the second case relates to a reception of the activation signal on the primary main window and the third case relates to a reception of the activation signal on a secondary window.

The realization of the invention by a computer program product and/or a computer-readable medium has the advantage that already existing servers, clients and/or computing entities associated to a medical device can be easily adopted by software updates in order to work as proposed by the invention. It is not necessary to amend the operating system.

The computer may operate in a networked environment which defines logical connections to one or more remote computers. The remote computer may be another personal computer, a server, a router, a network PC, a peer device or other common network node, and may include many or all of the elements described above relative to the personal computer and/or a medical system. The logical connections include a local area network (LAN) and a wide area network (WAN), an intranet and the Internet.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labeled with the same reference signs in different figures. In general, the figures are not for scale. It shall be understood that a preferred embodiment of the present invention can also be any combination of the dependent claims or above embodiments with the respective independent claim.

These and other aspects of the invention will be apparent from and elucidated with reference to the embodiments described hereinafter.
- Fig. 1: is a schematic representation of a multi monitor setting with a first and second application and its respective windows;
- Fig. 2: is a schematic representation of the multi monitor setting shown in Fig. 1 in which the secondary main window has been activated;
- Fig. 3: is another schematic representation of a single monitor setting with a first and a second application, wherein the first application has a primary and a secondary main window and wherein the second application has another window, which is activated;
- Fig. 4: is a schematic representation of the single monitor setting shown in Fig. 3 in which the primary main window has been activated;
- Fig. 5: is a schematic representation of the single monitor setting shown in Fig. 3 in which the secondary main window has been activated;
- Fig. 6: is an overview of the structure and architecture of a z-order adaption module according to a preferred embodiment of the present invention;
- Fig. 7: is a flow chart of a method for positioning windows according to a preferred embodiment of the present invention;
- Fig. 8: is a sequence diagram for representing the data ex-change between the respective entities according to a preferred embodiment of the present invention if the activation signal was received on a primary main window or on a dialog window;
- Fig. 9: is a sequence diagram for representing the data ex-change between the respective entities according to a preferred embodiment of the present invention if the activation signal was received on a secondary main window.

The invention aims at an improved positioning of windows in a multi-application and multi-window setting.

For placing windows, operating systems (e.g. WINDOWS) use a positioning mechanism (e.g. z-order mechanism), which is well established for a single application. But for a set of applications in the medical domain, where the importance of dialog windows is crucial, as they may relate to life-sustaining measures, the known z-order mechanism may lead to negative effects by hiding important dialog windows. This is the starting point for the invention.

In the following, the known z-order mechanism as used in WINDOWS is explained in more detail.

In general, in a graphical, e.g. Windows-based application, a window is a rectangular area of the screen where the application displays output and receives input from the user. Therefore, one of the first tasks of a graphical Windows-based application is to create a window. A window shares the screen with other windows, including those from other applications. Only one window at a time can receive input from the user. The user can use the mouse, keyboard, or other input device to interact with this window and the application that owns it.

The z-order of a window indicates the window's position in a stack of overlapping windows. This window stack is oriented along an imaginary axis, the z-axis, extending outward from the screen. The window at the top of the z-order overlaps all other windows. The window at the bottom of the z-order is overlapped by all other windows. The system maintains the z-order in a single list. It adds windows to the z-order based on whether they are topmost windows, top-level windows. A topmost window overlaps all other non-topmost windows, regardless of whether it is the active or foreground window. A topmost window has the WS_EX_TOPMOST style. All topmost windows appear in the z-order before any non-topmost windows. A child window is grouped with its parent in z-order.

The user may change the z-order by activating a different window. The z-order mechanism of the known operating system positions the active window at the top of the z-order for windows of the same type. When a window comes to the top of z-order, so do its child windows, all belonging to the same application. Problems may arise, if more than one application is operated in parallel.
An active window is the top-level window of the application with which the user is currently working. To allow the user to easily identify the active window, the system places it at the top of the z-order and changes the color of its title bar and border to the system-defined active window colors. Only a top-level window can be an active window. When the user is working with a child window, the system activates the top-level parent window associated with the child window.

Only one top-level window in the system is active at a time. The user activates a top-level window by clicking it (or one of its child windows), or by using the ALT+ESC or ALT+TAB key combination. An application activates a top-level window by calling the SetActiveWindow function.

When the activation changes from a top-level window of one application to the top-level window of another, the system sends a WM_ACTIVATEAPP message to both applications, notifying them of the change. When the activation changes to a different top-level window in the same application, the system sends both windows a WM_ACTIVATE message.

An overlapped or pop-up window can be owned by another overlapped or pop-up window. Being owned places several constraints on a window. An owned window is always above its owner in the z-order. The system automatically destroys an owned window when its owner is destroyed. An owned window is hidden when its owner is minimized. Only an overlapped or pop-up window can be an owner window; a child window cannot be an owner window.

As explained above, problems may arise in the medical domain with multiple applications running in parallel and wherein a specific handling and positioning of windows is necessary in order to safeguard the correct priority for different window requests.

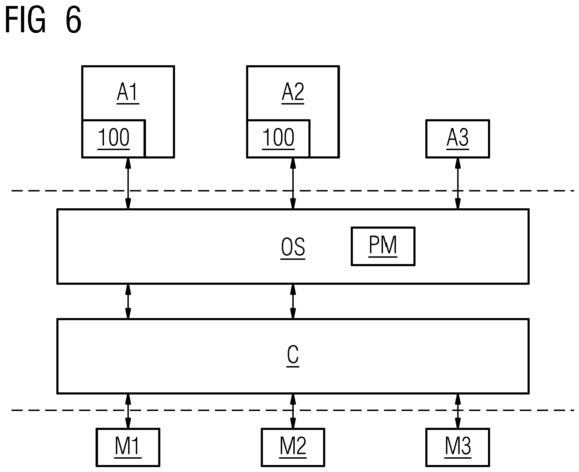

The general architecture in which the method and module according to the invention is integrated is explained with respect to Fig. 6.

Fig. 6 shows several (by way of example: three) different applications A1, A2, A3 which interact with the z-order adaption module 100 according to the invention. In Fig. 6 three layers are depicted: a first applicational layer with the applications A, a second layer relating to the operating system OS which may comprise a window manager and a controller system C for controlling the monitor hardware, and a third layer which relates to the hardware, with the monitor system M or M1, M2 and M3 respectively.

The z-order adaption module 100 is deployed as an instance on each application A, as can be seen in Fig. 6 with application A1 and S2. The instance of the z-order adaption module 100 may serve as an interface to the operating system OS, e.g. MICROSOFT WINDOWS with the window positioning mechanism PM, which is based on the z-order as described above. The window positioning mechanism PM instructs a graphic controller C (e.g. an external or internal graphic card), which controls the set of monitors M1, M2 by generating a feed of output images. User input is received on a window on one of the monitors M and will be communicated back to the applications A. If the application is a hosted application, the host application detects the creation of dialog windows by means of the "window creation detection system" and embeds the main windows of the hosted application as child windows in at least one main window of the host application.

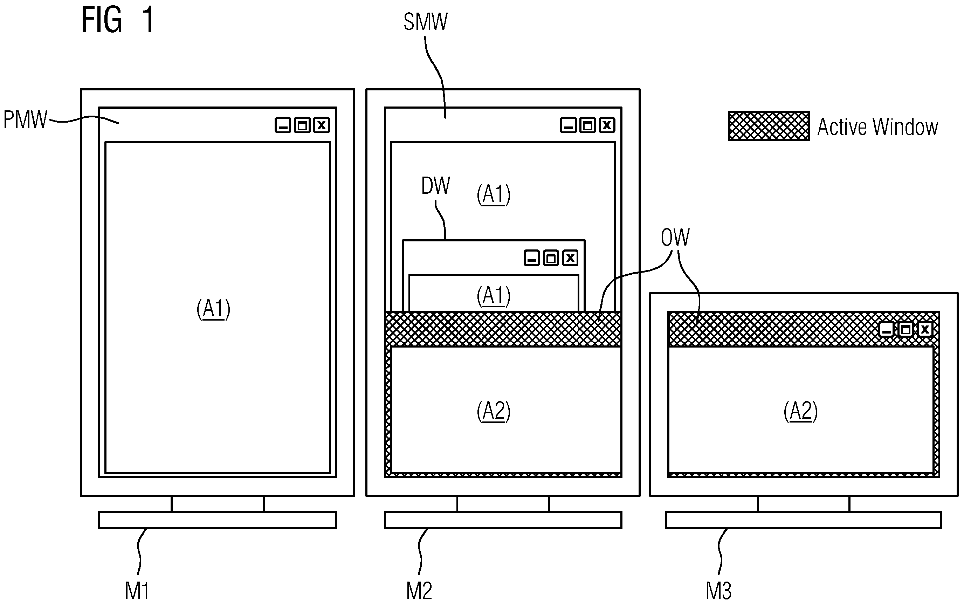

Fig. 1 shows a schematic representation of a multi monitor setting with three monitors M1, M2, M3 and with a first application A1 and second application A2 and its respective windows. The first application A1 generates two main windows, a primary main window PWW and a secondary main window SMW. The primary main window PMW of the first application A1 owns a dialog window DW. The windows of the first application A1 are positioned or moved to the first and second monitor M1, M2. The second application A2 may be of a third-party manufacturer and generates another window OW, which is depicted on the second and third monitor M2, M3. The window, which is currently used by the user and is therefore active is represented in the figures 1 to 5 with a dotted menu bar.

The z-order of the set of applications is: OW, DW, PMW, SMW. As can be seen in Fig. 1, the other window OW of the third-party application A2 partially hides windows of the first application A1.

If the user now wants to work with the first application A1, he may click on the secondary main window SMW.

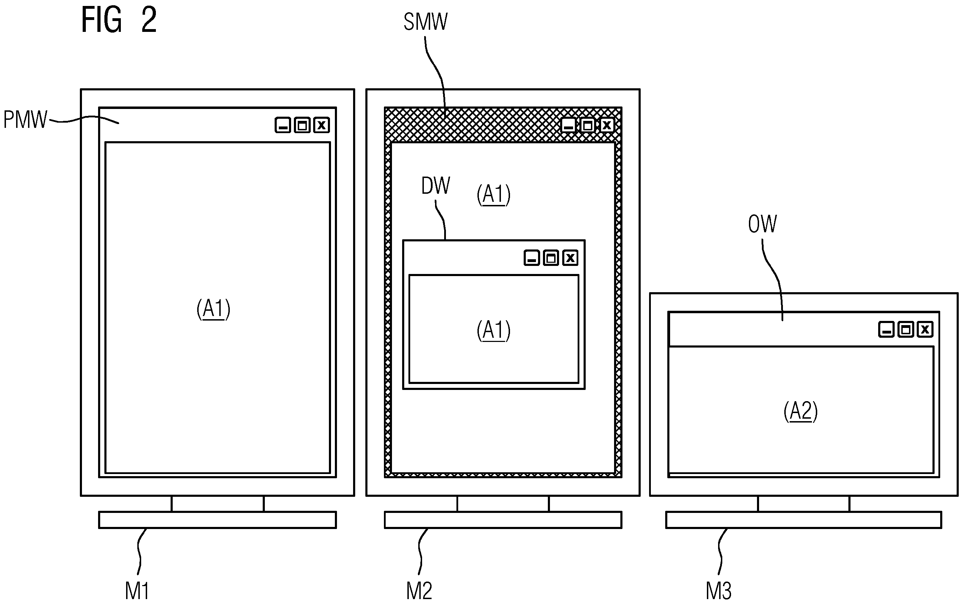

After receiving the user activation signal on the secondary main window SMW the method according to this invention and/or a z-order adaption module 100 generates a second control signal c2 for instructing the operating system's positioning mechanism PM to position the windows in the z-order sequence which is depicted in Fig. 2 from the front to the back: DW, SMW, PMW, OW. As can be seen, the original z-order (which would have been the result according to the normal operating system) is changed as usually the operating system's z-order - without applying the z-order adaption module 100 of the invention - would have been SMW, OW, DW, PMW.

It is to be noted, that the resulting positioning according to the invention is maintained even if the positions or locations on the screen of the primary main window PMW and the secondary main window SMW would be changed or reversed. Further, the resulting positioning is maintained even if the dialog window DW is positioned somewhere in front of the primary main window PMW on the first monitor M1 or on the third monitor M3.

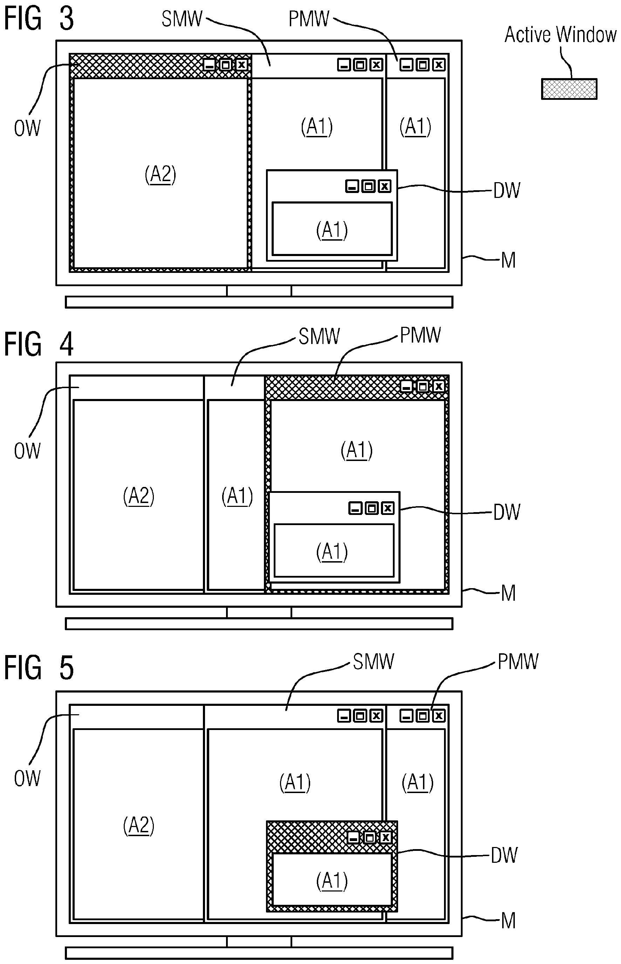

Fig. 3 shows another scenario with one single monitor M, which could be a scenario where a large scale monitor is used or with only one "normal" monitor M, because the other monitors are defect (emergency operation). The first application A1 has two overlapping main windows: a primary main window PMW and a secondary main window SMW. The first application A1 further generates a dialog window DW which is related to or owned by the primary main window PMW. A second application A2 creates another window OW, which is in the activated state, shown in Fig. 3 with dotted menu bar.

The z-order from the front to the back is: OW, DW, SMW, PMW. As can be seen in Fig. 3, the other window OW at least partially covers the windows SMW, PMW of the first application A1.

The user now wants to bring the application A1 (with its windows) to the foreground. He or she, therefore, clicks onto the primary main window PMW. This activation is represented in Fig. 4 - which shows the resulting positioning - with the dotted menu bar.

This activation signal is processed by the z-order adaption module 100, which instructs the operating system with an amended control signal, so that a resulting positioning will be executed, which is shown in Fig. 4.

The resulting z-order for the set of applications according to the invention is: DW, PMW, SMW, OW. All windows of the first application A1 are placed in front of the other windows OW of the second application A2. The primary main window PMW is placed in front of the secondary main widow SMW according to the user's intention. The dialog window DW is in front of all main windows of the application A1 and also A2.

Starting again at the scenario depicted in Fig. 3, the user now wants to work with the dialog window DW and activates the same. Then this activation signal is processed by the z-order adaption module 100, which again instructs the positioning mechanism MP of the operating system OS with an adapted control signal, so that a resulting (and amended) positioning will be executed, which is shown in Fig. 5. In Fig. 5, therefore, the dialog window DW is represented in dotted structure, because an activation signal was received thereon.

The resulting z-order for the set of applications according to the invention is shown in Fig. 5 and is: DW, SMW, PMW, OW.

In reply to the mouse click onto the dialog window DW and according to the positioning procedure, the dialog window DW is brought all the way to the front and in front of all other windows with respect to the z-order. By means of the owner-relation the primary main window PMW receives from the operating system the requirement to be placed directly behind the dialog window DW. Specifically, at this point, the invention amends the usual z-order. According to the procedure of the z-order adaption module 100, the order or request, which would usually be directed to the primary main window PMW is amended so that the secondary main window SMW is placed directly behind the dialog window DW instead of the primary main window PMW. The primary main window PMW is positioned behind the secondary main window SMW, so that no change of the relative z-order of the main windows takes place, but all come together to the foreground in relation to the other window OW.

In the scenarios shown in Fig. 2 and 5 the dialog window DW may be moved in front of another or foreign main window and not in front of their "own" primary main window (which owns them). The positioning mechanism PM nevertheless works well also in these settings.

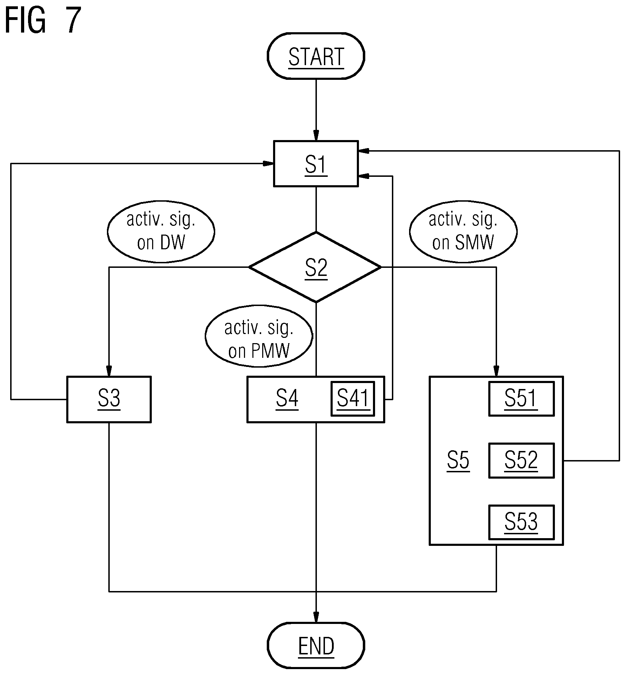

Fig. 7 shows a flow chart of the method for z-order positioning of windows. After start, in step S1 an activation signal is received, e.g. as user input on a graphical user interface element on the monitor M. In step S2 it is detected, whether the activation signal is received on the dialog window DW or primary main window PMW or secondary main window SMW. These three cases are represented in Fig. 7 in the different branches. If the activation signal is received on the secondary main window SMW, this case is represented in Fig. 7 in the right branch. Step S2 is executed by analyzing the signals which are sent from the operating system OS to the primary or secondary main windows PMW, SMW. The step S2 is thus not part of the z-order adaption module.

If the activation signal is received on the dialog window DW (left side), in step S3 messages exchanged between the application and the operating system are analyzed. This allows to detect all other dialog windows DW which do have the same owner relation as the activated dialog window DW. Based on this, a first control c1 signal is generated for positioning all such dialog windows as a dialog window package together with the dialog window, which has been activated in front of the primary main window PMW. The z-order adaption module 100 positions the secondary main window SMW behind or in front of the primary main window PMW in response to the first control signal c1 for PMW. All other windows OW of second or other applications Ai, if any, are positioned (by means of instruction of the first control signal c1) in the back and behind all other previously mentioned windows by instructions of the first control signal c1.

The step S3 is not part of the z-order adaption module 100, but is executed within the operating system OS, in particular within its positioning mechanism PM. Step S5 only generates one new control signal c3 to be sent to the secondary main window SMW, for positioning it with respect to the primary main window. However, step S4 and S5, which will be explained below, are.

If the activation signal is received on the secondary main window (right side), in step S5 a third control signal c3 is issued by the operating system OS and messages exchanged between the application and the operating system (in particular the positioning mechanism PM) are analyzed. In particular, in step S51 messages from the operating system's positioning mechanism PM to the secondary main window SMW are detected. Step S52 refers to triggering the primary main window PMW to be moved where the secondary main window SMW is requested, which moves all dialog windows DW to the front according to the operating system's positioning mechanism PM. Step S53 relates to amending an original secondary main window control signal so that the secondary main window SMW is moved in front of the primary main window PMW and after the dialog windows DW and insert all remaining secondary main windows SMW such that the overall z-order of all main windows except the notified secondary main window is retained. All dialog windows DW corresponding to or owned by the primary main window PMW are detected. With other words, the z-order adaption module 100 hence instructs the positioning of the primary main window PMW at the requested position of the secondary main window in response to the third control signal c3. The third control signal c3 for the secondary main window SMW is then amended by the z-order adaption module 100 to insert secondary main window SMW before primary main window PMW but after any dialog window DW. The third control signal c3 further initiates positioning any other windows OW, if any, behind the primary main window PMW.

If the activation signal was received on the primary main window PMW (in Fig. 7. represented in the middle /branch), a second control signal c2 is issued in step S2 for letting the positioning mechanism PM of the operating system OS execute, thereby detecting a primary main window change request. Within the detected primary main window change request: in step S41 all secondary main windows SMW are inserted after the primary main window PMW so that the order of the secondary main windows SMW is retained and that the primary main window PMW is on top of the secondary main windows SMW.

After the steps S3, S4 or S5 the method may end or may be reiterated as shown in Fig. 7.

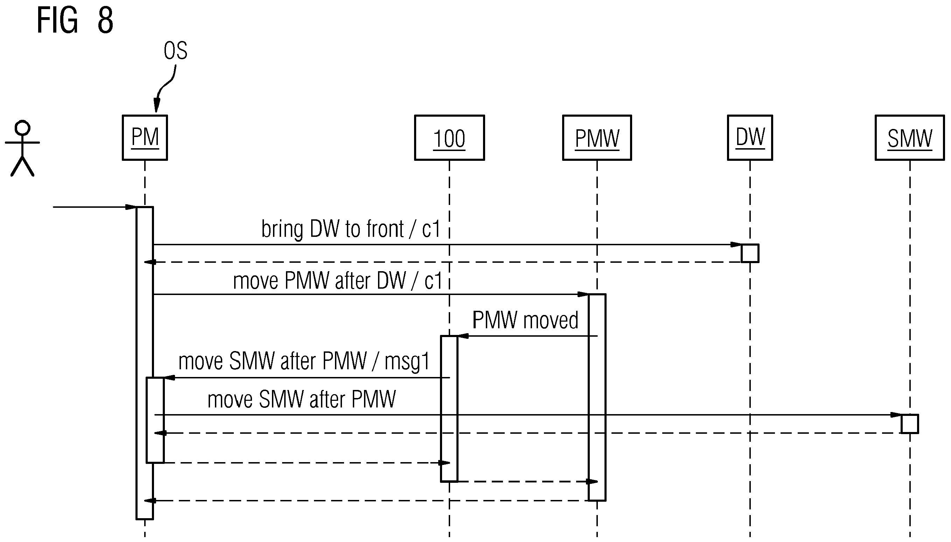

Fig. 8 is an interaction diagram representing the message transfer between the application A with the z-order adaption module 100 deployed therein, the operating system OS, in particular with the positioning mechanism PM thereof, and the monitor M for displaying the primary main window PMW, the secondary main window SMW and dialog windows DW. After detection that the activation signal was received on a primary main window PMW or on a dialog window DW, the operating system OS instructs the dialog window DW to be placed in the front by the control signal c1 "bring DW to front". The corresponding primary main window PMW is instructed by the operating system OS to be moved behind the dialog window DW with the instruction of the control signal c1 "move PMW after DW". If the primary main window PMW has been moved a respective message "PMW moved" is transferred to the z-order adaption module 100 which in turn transmits a message msg1 with a request to "move SMW after PMW" to the operating system OS which in turn notifies and moves the secondary main window SMW to the requested position. As can be seen the z-order adaption module 100 produces additional signals in response to the first control signal c1 to instruct the respective window positioning actions. The number is increased if more than one secondary main window is present.

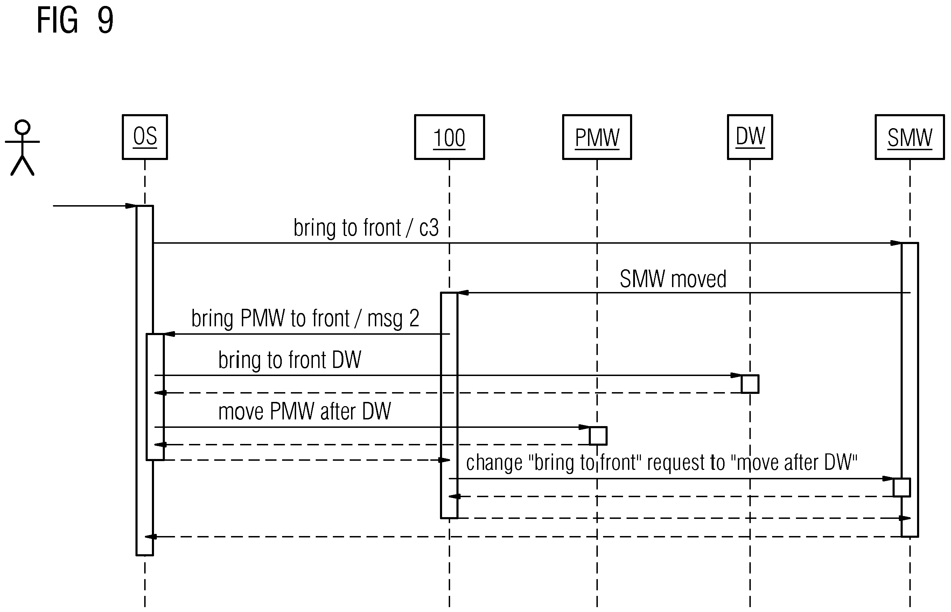

Fig. 9 shows an interaction diagram representing the message transfer between the application A with the z-order adaption module 100 deployed therein, the operating system OS, in particular with the positioning mechanism PM thereof, the primary main window PMW, the secondary main window SMW and dialog windows DW. After detection that the activation signal was received on a secondary main window SMW, the operating system OS instructs the secondary main window SMW by means of the third control signal c3 with the instruction "bring to front", which in reply sends a message as a notification to the z-order adaption module 100 "SMW moved". Then the z-order adaption module 100 generates a second message msg2 with the instruction "bring PMW to front" and sends the same to the operating system OS, which in reply generates and sends the respective instructing signals to the windows DW and PMW. In Fig. 9 on the left part with respect to the operating system OS, there are depicted two different (rectangular) execution blocks, which should represent the fact that from the perspective of the operating system OS the instruction "bring PMW to front" is a new signal, which is indirectly calculated on the basis of the third control signal c3. As can be seen, the positioning mechanism PM sends multiple (i.e. two) messages to instruct the respective windows and the z-order adaption module 100 sends another message "change 'bring to front' request to 'move after DW'" to change or amend the original operating system's positioning instruction.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

The system for controlling a z-order positioning of windows in accordance with the method as described above can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

The invention may be used in the medical domain and in particular for systems of the applicant like syngo.via, syngo.via View&Go, all diagnostic applications, scanner and therapy applications based on native syngo or its modules and/or to all multi monitor enhanced applications, which may be based on the Windows operating system.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantageous which are described with respect to a particular embodiment of present invention or with respect to a particular figure are, wherever applicable, also advantages of other embodiments of the present invention.

## Claims

1. Computer-implemented method for controlling a positioning of more than one window with respect to a z-order for at least one application (A1, A2), running on a computer with a monitor system (M1, M2, M3), wherein the at least one application (A1) has a primary main window (PMW) and at least one secondary main window (SMW), wherein the primary main window (PMW) may own a set of dialog windows (DW), wherein the method reacts on the request to bring any of the multitude of windows to the foreground of the monitor system (M1, M2, M3) with a request to bring the dialog windows (DW) and the main windows to the foreground, thereby ensuring that the dialog windows (DW) are always in front of any of the primary and secondary main windows (PMW, SMW), and wherein the z-order of the primary and secondary main windows (PMW, SMW) can change within the application (A1), which ensures that the z-order of overlapping primary and secondary main windows (PMW, SMW) can be altered in a way that its content can be made visible and wherein primary and secondary main windows (PMW, SMW) of the application (A1) are contiguous within a desktop window list which ensures that no other window (OW) of any other application (A2) appears mixed with the application (A1).

2. Method according to claim 1, wherein the method comprises the method steps of:
- Receiving (S1) an activation signal on a window;
- Detecting (S2) whether the activation signal was received on a dialog window (DW), owned by the primary main window (PMW) or on a primary main window (PMW) or on at least one secondary main window (SMW);
- If the activation signal was received on a dialog window (DW) :
Issue (S3) a first control signal (c1) for letting the positioning mechanism (PM) of the operating system (OS) execute, thereby detecting a primary main window change request and within the primary main window change request: inserting all secondary main windows (SMW) in front of or after the primary main window (PMW) so that the order of the primary and secondary main windows (PMW, SMW) is retained;
- If the activation signal was received on the primary main window (PMW):
Issue (S4) a second control signal (c2) for letting the positioning mechanism (PM) of the operating system (OS) execute, thereby detecting a primary main window change request and within the primary main window change request: inserting (S41) all secondary main windows (SMW) after the primary main window (PMW) so that the order of the secondary main windows (PMW, SMW) is retained and that the primary main window (PMW) is on top of the secondary main windows (SMW);
- If the activation signal was received on at least one secondary main window (SMW):
Issue (S5) a third control signal (c3) for:
- Detecting (S51) messages from the operating system's positioning mechanism (PM) to the secondary main window (SMW);
- Triggering (S52) the primary main window (PMW) to be moved where the secondary main window (SMW) is requested, which moves all dialog windows (DW) to the front according to the operating system's positioning mechanism (PM) and for
- Amending (S53) an original secondary main window control signal so that the secondary main window (SMW) is moved in front of the primary main window (PMW) and after the dialog windows (DW) and insert all remaining secondary main windows (SMW) such that the overall z-order of all main windows except the notified secondary main window is retained.

3. Method according to any of the preceding claims, wherein the method is executed as an instance in or by the application (A) and comprises to intercept the messages between the application (A) and a z-order positioning mechanism (PM) of the operating system (OS) without changing the operating system (OS).

4. Method according to any of the preceding claims, wherein issuing the third control signal (c3) causes the primary main window (PMW) and subsequently the dialog windows (DW) to be moved to the front in the z-order although they are not related through the owner relation with the corresponding secondary main window (SMW) which has been activated.

5. Method according to any of the preceding claims, wherein the monitor system (M) is a single monitor or a multi-monitor system.

6. Method according to any of the preceding claims, wherein the activation signal is effected by a user interaction on a window, by an activation of a key and/or by a functional request of an application transmitted via an application programming interface/API.

7. Method according to any of the preceding claims, wherein at least one third-party application (A2) is hosted within the operating system (OS) in addition to the application (A1), and wherein the third-party application (A2) has at least one main window, which is/are hosted by adding it/them as child windows to main windows of the hosting application (A1) and wherein dialog windows of the third-party application are detected and added as dialog windows to the hosting application's primary main window (PMW).

8. Method according to any of the preceding claims, wherein separate applications can be hosted by the application (A1) and a window creation detection system is used, which detects any newly created window for the hosted applications that changes a dialogs owner relation to the primary main window (PMW) of the application (A1).

9. Method according to any of the preceding claims 2 to 8, wherein a bring-to-front-request is overwritten during processing the third control signal (c3), if the activation signal was received on at least one secondary main window (SMW), in particular changed to a move-after-dialog-window request.

10. Method according to any of the preceding claims, wherein at least one dialog window (DW) may be moved by user interaction out of its original position and at least in part out of a region of its primary main window (PMW).

11. Method according to any of the preceding claims, wherein although the primary main window (PMW) and the secondary main window (SMW) and optionally other windows (OW) may at least partially overlap or may be displayed on separate monitor devices of the monitor system, it is assured that the dialog windows (DW) are displayed at a z-order position so that they are never covered by any part of a MW.

12. Method according to any of the preceding claims, wherein the secondary main window (SMW) does not own one or more dialog windows (DW).

13. Method according to any of the preceding claims, wherein the secondary main window (SMW) belongs to the same application as the primary main window (PMW).

14. Method according to any of the preceding claims, wherein the z-order of the whole set of dialog windows (DW) of the application is changed in common and as one and in reply to where the activation signal was detected.

15. A z-order adaption module (100), wherein an instance of the z-order adaption module is deployed in or used by an application, which generates content, in particular medical content, to be displayed in windows of a monitor system, so that the application is adapted to apply a method according to any of the preceding method claims.

16. A computer program comprising program elements which induce a computer to carry out the steps of the method for controlling a window z-order positioning according to one of the preceding method claims, when the program elements are loaded into a memory of the computer.
